# EUROPEAN PATENT SPECIFICATION

(11) **EP 3 131 619 B1**
(45) Date of publication and mention of the grant of the patent: **19.07.2023**
(21) Application number: 15779197.1
(22) Date of filing: 16.04.2015
(51) Int. Cl.: A61N 1/00, A61H 19/00

(54) **STIMULATING DEVICES**
STIMULATIONSVORRICHTUNGEN
DISPOSITIFS DE STIMULATION

(30) Priority: 16.04.2014 US 201461980161 P
(43) Date of publication of application: 22.02.2017
(73) Proprietor: Hamilton, Christopher, Chad, Kirkland, Washington 46525 (US)
(72) Inventor: Hamilton, Christopher, Chad, Kirkland, Washington 46525 (US)
(74) Representative: Haseltine Lake Kempner LLP
(86) International application number: PCT/US2015/026110
(87) International publication number: WO 2015/161038

(56) References cited:
- WO-A1-2013/063420
- WO-A1-2014/043263
- WO-A2-2008/088985
- US-A1- 2003 114 902
- US-A1- 2007 088 403
- US-A1- 2007 191 911
- US-A1- 2008 027 507
- US-A1- 2008 027 507
- US-A1- 2010 004 715
- US-A1- 2010 004 715
- US-A1- 2012 089 059
- US-A1- 2013 158 627

## Description

### BACKGROUND

This innovation relates in general to electronic devices, and more specifically to stimulating electronic devices including various stimulating elements.

The present invention is set out in the appended claims. WO 2008/088985, WO 2013/063420 and US 2010/004715 describe different stimulating devices.

### SUMMARY

This innovation generally relates to a stimulating components engaged or disengaged by various controllers. In an embodiment, a stimulating device includes a flexible board configured for personal use, an array of one or more vibrational stimulating components disposed throughout at least a portion of the flexible board, a stimulation controller in communication with at least one of the one or more vibrational stimulating components, the stimulation controller configured to process a stimulation signal that activates or deactivates at least one of the one or more vibrational stimulating components, at least one sensor integrated into the flexible board for collecting body feedback data, and a wireless radio in communication with the stimulation controller and configured to receive the stimulation signal wirelessly from at least one remote device, wherein the controller is further configured to modify the stimulation signal based on body feedback data.

In further aspects of the innovation, there can be a non-therapeutic method comprising processing a stimulation signal that activates and deactivates one or more vibrational stimulating components integrated into an array within a flexible board configured for personal use; activating and deactivating the one or more vibrational stimulating components and collecting body feedback data using at least one body feedback sensor (250) integrated into the flexible board, wherein the stimulation signal is received using a wireless radio operatively coupled with the flexible board.

Optional features of the invention are set out in the dependent claims.

Various aspects will become apparent to those skilled in the art from the following detailed description and the accompanying drawings.

### BRIEF DESCRIPTION OF THE DRAWINGS

Figs. 1A, 1B, and 1C illustrate flexible boards having arrays of stimulating components;
Figs. 2A, 2B, and 2C illustrate functional block diagrams of stimulating devices;
Fig. 3 illustrates a functional block diagram of a stimulating signal being broadcast to a plurality of stimulating devices;
Fig. 4 illustrates an example computing environment relating to aspects herein;
Fig. 5 illustrates a method for stimulation using stimulating devices; and
Fig. 6 illustrates a method for broadcasting a stimulation signal to a plurality of stimulation devices.

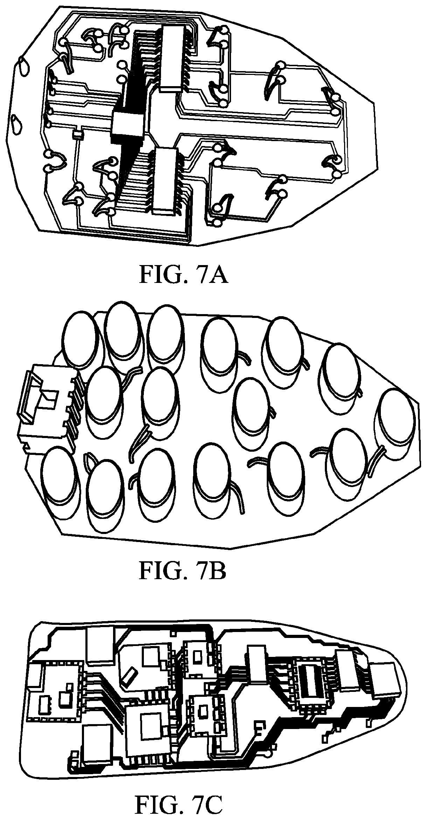

Figs. 7A, 7B, and 7C show systems and components in accordance with disclosures herein.

### DETAILED DESCRIPTION

The innovation is now described in various aspects. In the following description, for purposes of explanation, numerous specific details are set forth in order to provide a thorough understanding of the subject innovation. It may be evident, however, that the innovation can be practiced without these specific details. In other instances, well-known structures and devices are shown in block diagram form in order to facilitate describing the innovation. While one or more drawings accompanying this detailed description may or may not be discussed, all aspects from the drawings are wholly incorporated herein, as are any other appendices or supplemental materials. Where materials provided herewith conflict regarding scope of the subject innovation, such conflicting aspects should first be read as alternative or complementary embodiments, and if no such reading is possible, the broader reading shall apply. The present invention is set out in the appended claims. Any embodiments, examples or aspects according to the present description which do not fall within the scope of said claims are provided for illustrative purposes only.

Aspects of the innovation disclosed herein include a new form of massager. The massager can localize vibration activity, and vary the intensity of vibration at one or more locations. Touch can be simulated using various feedbacks at various locations.

The device or devices may, for example, be integrated into clothing, attached to clothing, or attached to the body. Other means of permitting systems to interact with persons will be appreciated on review of the disclosures herein.

Systems and methods herein utilize, for example, modern compact vibrator and haptic feedback components. Moving components can be powered by driver components, which in turn can be controlled by a microcontroller. In some embodiments, there are also other solenoid or electromagnetic or electro-mechanical devices which can, e.g., cause larger movement. In embodiments, other components can be controlled, or have their function augmented, directly or through intervening components.

As used herein, a "flexible board" is a flexible or semi-flexible component capable of connecting stimulating components into a single device. A flexible board in this regard can be similar to a circuit board inasmuch as it is a common mechanical support or substrate for a plurality of electronic components. While "board" imputes a planar arrangement having predictable dimensions and shapes, this need not be the configuration for flexible boards herein. For example, dimensions such as height, length, and thickness (and/or depth through a portion thereof) can taper or flare. Portions or the entire board can be rounded or of any number of similar or dissimilar sides.

The material of the flexible board's substrate can be elastic or inelastic in nature. In an embodiment, the flexible board may be bent or shaped but return to its original shape or configuration (planar, curvilinear, *et cetera)* upon release of the shaping forces. In an alternative embodiment, the flexible board may remain in its configuration until re-shaped, thereby having a "memory" characteristic or flexible layer that remains in a formed shape until again modified. Flexible board designs can further be stretchable or pliable, permitting deformation in more than one dimension.

The portion of the flexible board providing stimulation and activity onto the user can be based on fiberglass flexible printed circuit boards, silicone-based designs, and others. Other materials can include, but are not limited to, polyimide, polyester, and other polymers. In embodiments, conductive silicone can be used and integrated with output devices and circuit boards.

One or more components, including the pad itself, can utilize silicone or similar materials (such as, *e*.*g*., fluorosilicone, ethylene propylene diene monomer, fluorocarbon, various rubber-type materials). In embodiments, the flexible board is formed at least in part of a flexible conductive material. Conductive elastomers can be used in various embodiments, and can be used to prevent moisture intrusion into electrical/electronic components, shield components from pressure, and protect components from electromagnetic interference. Conductive elastomers can integrate conductive particles (*e*.*g*., metal fillers), utilize various plating techniques, and/or leverage other options for creating one or more conductive paths through a flexible material. In embodiments using conductive material, conductive silicone or alternatives can be utilized.

In at least one alternative embodiment, a board herein can be non-conductive, and in another alternative embodiment, non-flexible. In an embodiment, a subordinate board may be integrated with the flexible board, such as the inclusion of a rigid board operatively coupled with the flexible board. Rigid portions can include softening characteristics, such as coating with silicone, neoprene, or other materials. These materials can also provide strain and load relief or reinforcement to the flexible board, connection points on the flexible board (*e*.*g*., solder joints or epoxy application areas), or modules or components attached at such connection points. Another benefit of such materials is provided in the form of isolation of components to prevent vibration or other forces at one location in or on a flexible board from impacting stimulating components or sensors at other locations in or on the flexible board. In at least one embodiment, voids can be included in coatings or structural flexible materials to enhance the isolation and independence of components in or on the flexible board. Motors or other aspects used in conjunction with stimulating components can be wired below flexible or softening layers.

To facilitate flexibility, waterproofing, and comfort, conductive rubber o-rings can be crimped into or otherwise operatively coupled with flexible boards. These can be used with, for example, connector pins to mate with one or more wire gauges. In an embodiment, pins are inserted into a connector header and receptacles attached on the flexible board, and rubber can be wrapped on motor leads (to motor drivers) to place motors along the o-ring diameter. O-rings can be stacked or layered in one or multiple sizes, and multiple layers and sizes of o-rings can be used to add more motor and/or driver lines in such embodiments.

Inasmuch as aspects herein are described to be "configured for personal use," such aspects are designed or arranged to be worn by or applied to an individual, or intimately shared between individuals. While discussion of intimate sharing imputes sexual activity, aspects herein need not be limited to such context. Instead, intimate application may be used to describe relatively close contact. In embodiments, stimulation components can be utilized for foot massage or placed at or around acupuncture points on non- or less-sexual regions of human anatomy. Aspects can be arranged to conform to or mate with various aspects of human anatomy, and can be configured for discreet use in conjunction with regular clothing or specific clothing or entertainment accessories. In at least one embodiment, durable "wings" can be provided at the edges of a flexible board. Such "wings" can include various attachment means, such as adhesives or hook-and-loop fasteners, to facilitate attachment to underwear or other clothing items. An example construction in this regard can include a flexible board with wings having rigid modules surrounded by flexible or softening materials operatively coupled (*e*.*g*., attached, soldered, connected) thereto.

Rigid modules described herein can include rigid boards, power supplies, universal serial bus ports or components, Bluetooth^{®} components or modules, microcontrollers with or without support for digital signal processing and/or analog-to-digital converters, various electrodes (*e*.*g*., for electrocardiography or other purposes), position sensors or boards (*e*.*g*., 12-axis position board), flex or force sensors, other sensors and/or sensor input modules, and others.

As used herein, "stimulating components" are components capable of imparting sensory stimulation to living entities. Aspects herein focus predominantly on touch, providing massaging or vibrating capabilities. However, nothing herein should be construed as limiting stimulating components from including components capable of emitting sound, visual stimulation, and/or detectable smells and tastes. Further, aspects of touch other than massaging or vibrating can be utilized, including (but not limited to) electrical stimulation, temperature stimulation, and others.

Stimulating components can be vibrators, haptic feedback modules, motion devices, motors, air compressors or other elements capable of producing blowing or suction, *et cetera.* Solenoids and/or other electromagnetic or electromechanical aspects can be employed. Vibrators, haptic modules, and motion devices can include resonant actuators, and such as linear resonant actuators. In embodiments, sizes of such actuators can range between 2mm and 20mm, although these dimensions are given for purposes of example rather than requirement. True haptic actuators can be used to provide detailed haptic feedback effects. Linear resonant actuators can provide different behaviors when compared to eccentric rotating mass vibrators/vibration motors. Various combinations of different motor/vibrator types can be employed to effect different feedback. Moving parts can have different vibration amplitudes, ranges of motion, power settings, and so forth.

Engagement or disengagement of stimulating components can include energizing or deactivating the components. Deactivation can include ceasing activity in a stimulating component or returning a stimulating component to a baseline or background activity level. Engaging and disengaging stimulating components can thus be binary in nature, *e*.*g*., "on" or "off." Alternatively, engagement of a stimulating component can be conducted across a spectrum or scale of activity, including various different modes or intensities at which engagement occurs. In an embodiment, engaging a stimulating component can energize the stimulating component at one of several intensities (*e*.*g*., discrete settings or a continuous scale of settings). Intensity can include, for example, force or speed. Further, different modes can include continuous activation or engagement, or various patterns of pulsing or varied engagement. For example, linear resonant actuator motors can brake movement to provide precise vibration patterns and schemes. Other examples of stimulating components relating to vibration or movement can include common eccentric rotating mass vibration motors, electromagnetic solenoid movement components, Piezo vibrators, and various other haptic feedback components (including but not limited to ultrasonic haptics).

As used herein, an "array" can be any arrangement of one or more components. Arrays on flexible boards are disposed about or on the flexible board. A three dimensional (3D) array, in this regard, is an array distributed through three axes, meaning at least one element of the array is disposed through each of length, width, and depth (*i.e.,* not all elements are disposed on a single plane). Arrays can be symmetrical or asymmetrical and/or can be arranged in patterns or randomly. Elements of arrays can act individually or coordinate activity between various elements to provide various patterns or schemas of use *(e.g.,* in the case of stimulating components, mimicking movement or other activity across an area or through a volume larger than a single stimulating component). As will be appreciated in the figures, coordinate systems can be defined in relation to a length (*x*), width (*y*), and/or depth (*z*) of the figures. As suggested, component and/or material dimensions or arrangements can vary over the layout of the array (*e*.*g*., narrowing or widening, changing directions). Arrays can also include a single type of stimulating component or two or more types of stimulating components. Non-limiting examples of arrays can include a four-by-four grid, a diamond-shaped arrangement of stimulating components or sensors (*e*.*g*., outline-only or filled with evenly-spaced stimulating components), a circle of ten elements, a random arrangement of 20 stimulating components in a cube, *et cetera.* In addition, arrays can have concentrations at points or in areas/volumes, *e*.*g*., less dense arrangement of array elements at periphery of concentrated area containing an increased number of elements per unit area/volume. For example, a concentration of stimulating components can be focused at an acupuncture point with a less dense arrangement of stimulating components arranged around the acupuncture point. In an embodiment, the density of stimulating components, sensors, or other array elements can be dependent upon a distance (*e*.*g*., radial distance) from a point or points.

As used herein, a "body feedback sensor" is a sensor used to collect feedback from a user or manual-controller of a stimulating device herein. Body feedback sensors can include, but are not limited to, thermometers, heartbeat monitors, perspiration or galvanic response sensors, breathing monitors, eye movement monitors, brain wave monitors, muscle or electromyography sensors, humidity or moisture sensors, and others. Gyros or accelerometers, ultrasound imaging or analyzing components, barometers, and other sensors can also be employed. In still further embodiments electromagnetic fields, chemical monitors, sensors tracking conductivity, resistivity, and/or capacitance, and/or other techniques for monitoring body chemistry, can be included in body feedback sensors. The sensors or components operatively coupled therewith can obtain, save, and/or transmit the data related to information collected by a sensor, or otherwise make data related to information collected by a sensor available to other components of systems and techniques herein.

Sensors measuring aspects not directly related to a human user or operator body can also be included in systems or used by methods described herein. For example, sensors measuring position, motion, flex, pressure, and other forces or variables on a stimulating device can be included despite the fact that such forces or variables are not necessarily quantities or qualities of the human user or operator. Thus, while portions of this disclosure relate primarily to body feedback sensors, such terminology should not be interpreted as limiting or precluding use of other sensors related to the systems or methods themselves. Where body feedback sensors are discussed, the spirit and scope of this innovation embrace the use of such other sensors as well.

"Operatively coupling" used herein describes interaction of components which act upon one another or communicate (one-way, two-way, or through/involving additional components). Such action can be accomplished through mechanical interaction of solid components which are directly connected or which exert forces on one another through various linkages or at a distance, or through the transmission of electricity or electrical signals through conductive media or wirelessly over the air. Such action can also be accomplished through fluid communication, which can be effected directly or through the direction of fluid matter through intervening or connecting components. These are only examples, and should not be construed as limiting or preventing the means by which components (both physical and logical) can interact in systems and methods described herein. In this regard, a stimulation component or sensor embedded in a flexible board is operatively coupled with the flexible board; and a first wireless radio and a second wireless radio are operatively coupled where the first wireless radio is embedded in a flexible board unattached to the second wireless radio but receives signals from the second wireless radio.

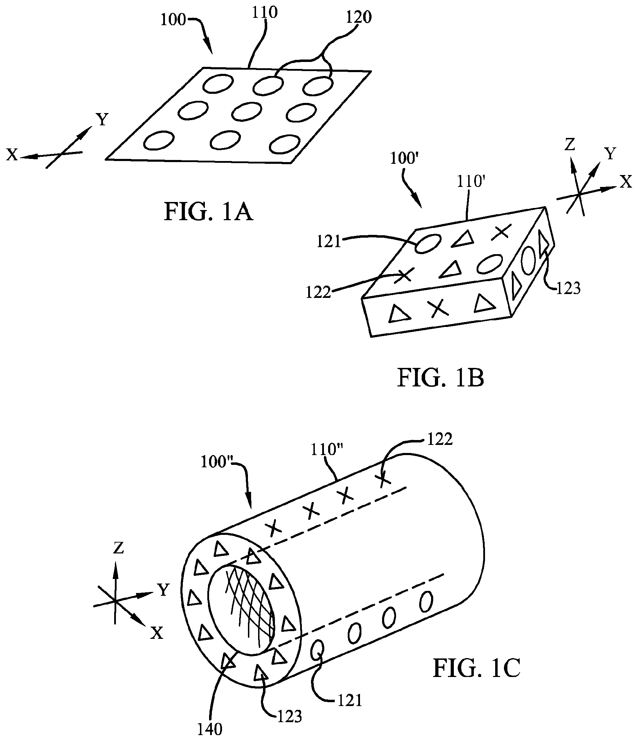

Now turning to the figures, Figs. 1A, 1B, and 1C depict various embodiments of a stimulation device 100 disclosed herein. Flexible board 110 provides connective material between stimulating components. In various embodiments stimulation device 100 may stimulate a user sexually. In this manner, stimulation device 100 may penetrate a body, or may touch a body without penetration. Stimulation device 100 may flex to fit a body, and/or be worn discreetly separate from or integrated with clothing items.

Fig. 1A illustrates an embodiment of a stimulation device 100 in which all stimulating components 120 are substantially disposed or arranged in a common plane. While stimulating components 120 are shown in a symmetrical array, various other arrangements are also embraced in this and other embodiments. For example, in Fig. 1A, in Figs. 1B and 1C, and in other embodiments described herein, asymmetrical arrangements or patterns can be utilized.

Further, stimulating components 120 (or stimulating components of other embodiments) can be concentrated or organized in multiple arrays or patterns. These can be based on position (within flexible board 110 or with respect to a user), size (of flexible board 110, other components, or a user), and/or human anatomy. In the same regard, portions of flexible boards herein can have few or no stimulating components.

Fig. 1B illustrates an embodiment of a stimulation device 100' having stimulating components 121, 122, and 123 disposed through a thickness and/or depth of flexible board 110'. Stimulating components 121, 122, and/or 123 are not all arranged in a common plane, and are distributed throughout the length, width, and depth of flexible board 110'.

As suggested, stimulating components in stimulation device 100' (or other embodiments depicted herein) can be different stimulating components. In an embodiment, at least two of stimulating components 121, 122, and 123 are distinct (*e*.*g*., mechanically, electronically, or otherwise) in design. In at least one embodiment, distinct or matching stimulating components 121, 122, and/or 123 can receive instructions from different or multiple controllers (*e*.*g*, stimulating components 121 coupled with a first stimulating controller, stimulating components 122 coupled with a second stimulating controller, and stimulating components 123 coupled with the first and second stimulating controllers). However not all embodiments require such and in at least one embodiment stimulating components 121, 122, and/or 123 can be substantially identical.

Fig. 1C illustrates an embodiment of a stimulation device 100" having stimulating components 121, 122, and 123 disposed through a thickness and/or depth of flexible board 110". In addition to depicting multiple stimulating components 121, 122, and 123 distributed through the length, width, and depth of flexible board 110", Fig. 1C further illustrates shaping of flexible board 110". Because board 110" is flexible, board 110" can be shaped to a cylinder or other shape accommodating human anatomy. In the illustrated embodiment, the hollow cylindrical arrangement of flexible board 110" can be configured to accommodate, for example, at least one male sexual organ. Further, flexible board 110" (or other flexible boards herein) can be further rolled or twisted, unrolled, bent, re-shaped, or otherwise flexibly modified to alternative configurations.

In at least one alternative embodiment, at least a portion of a board can be non-flexible but formed to a particular shape. In this regard, the non-flexible board can be coated, lined, or surrounded with a soft or flexible buffer material for user comfort.

While Figs. 1A, 1B, and 1C depict embodiments having relative sizes, proportions, and dimension ratios, other sizes, proportions, dimensions, *et cetera* are embraced within the scope of this disclosure. Further, flexible boards can be formed as any one or more of two- and three-dimensional shapes such as triangles, rectangles, circles, cuboids, spheres, rings, cones, prisms, cylinders, and others. Such arrangements can be solid or have one or more openings of one or more sizes at any position therein. Such openings can be through a portion or the entire depth of the board.

In an embodiment, flexible boards can be configured to accommodate male or female sexual organs, and are arranged for application to one or more surfaces thereof, and/or acceptance of or insertion into the same. Alternatively or complementarily, one or more flexible boards can be integrated into clothing, accessories, or other wearable items. In this regard, multiple flexible boards can be used with a stimulating device such that a distributed arrangement can be applied to one or more users or manual controllers for coordinated or individual use.

Still further, various embodiments provide that one or more stimulating components be engaged or disengaged based on relative or absolute positions of the stimulating components and/or human anatomy with which they are in contact.

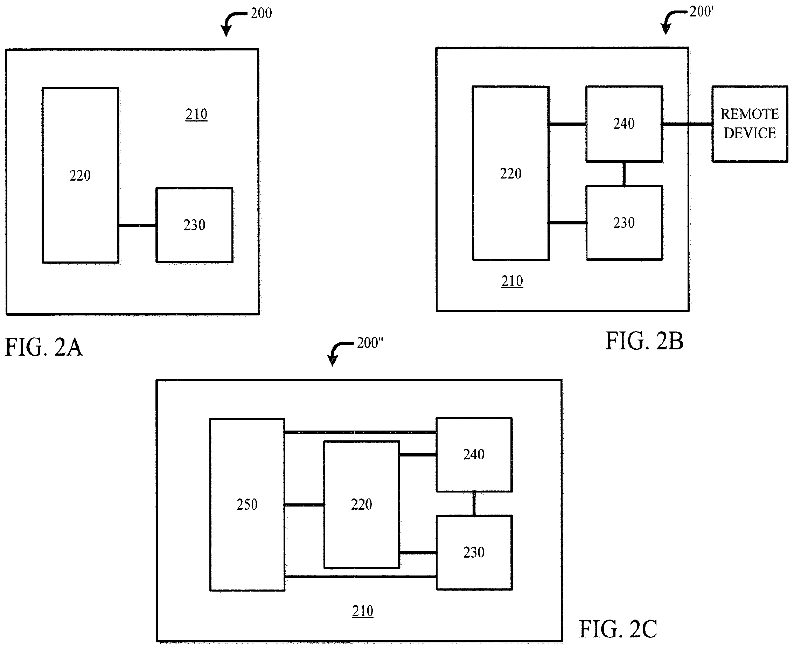

Turning to Figs. 2A, 2B, and 2C, block diagrams illustrating stimulating devices are depicted. Fig. 2A depicts stimulating device 200 having controller 230 and stimulating component array 220 disposed on flexible board 210. Controller 230 is operatively coupled with one or more stimulating components of stimulating component array 220. The stimulation controller processes a stimulation signal that engages or disengages at least one of the stimulating components. The stimulation signal can be programmed into controller 230, stored in local or remote memory, or provided through a remote or other device operatively coupled with controller 230. In an embodiment, controller 230 (or other components) provide one or more stimulation schedules for engagement of one or more stimulating components of stimulating component array 220 based on the stimulation signal.

Power can be provided using, *e*.*g*., batteries or other devices. Proprietary or custom curved battery or power supply shapes, or flexible batteries or power supplies, can be utilized to contour with a pad or other stimulation device. In an embodiment, a curved, moisture-resistant, and/or flexible battery holder which accepts common battery sizes can be integrated into a stimulating device. In at least one embodiment, rigid prismatic batteries can be utilized in arrays to allow for flexibility or non-standard shaping of a power supply in a stimulating device. Alternatively or complementarily, proprietary or custom batteries can be recharged or replaced. In still further embodiments, the device may use USB, inter-integrated circuits (i2c), or other combined communication and power connections or ports for power. In such embodiments, USB power can be passed to other USB peripherals where a stimulating device acts as a host or USB On The Go.

Fig. 2B illustrates another embodiment of stimulating device 200' having stimulating component array 220 operatively coupled with controller 230 on flexible board 210. Stimulating device 200' further includes wireless radio 240 operatively coupled with at least one of controller 230 and stimulating component array 220. Wireless radio 240 communicates with, *e.g.,* a remote device and receives at least a portion of a stimulation signal therefrom. The stimulation signal is thereafter applied to one or more elements of stimulating component array 220 to engage and/or disengage said elements.

Stimulating signals may be produced, modified, or sent (*e*.*g*., transmitted, received, energized, deactivated) using, *e.g.,* a handset such as a wireless telephone, proprietary remote, or other communication device. Remote devices can be assigned in a 1-to-1 manner (*e*.*g*., one device or remote controls a single stimulation device) or in a broadcast manner wherein a remote device may control multiple stimulation devices.

In at least one embodiment, stimulation signals can be synced to media. For example, the beat of a song, certain visual characteristics within a video, or other qualities or quantities within media can cause one or more stimulating components to be energized or deactivated. Put another way, media can be interpreted in terms of physical feedback in a manner that combines music, video, and other media with, for example, vibration patterns and location. Such control can be pre-programmed to match media, or extrapolated from the media based on processing in real-time.

Fig. 2C illustrates another embodiment of stimulating device 200" having stimulating component array 220 operatively coupled with controller 230 on flexible board 210. Stimulating device 200" also includes wireless radio 240 operatively coupled with at least one of controller 230 and stimulating component array 220. Wireless radio 240 communicates with, *e.g.,* a remote device and receives at least a portion of a stimulation signal therefrom. The stimulation signal is thereafter applied to one or more elements of stimulating component array 220 to engage and/or disengage said elements.

In stimulating device 200", wireless radio 240 is a transceiver capable of sending and receiving information. Stimulating device 200" further includes body feedback sensor(s) 250 which collect body feedback from one or more users of stimulating device 200". Body feedback sensor(s) 250 are integrated into flexible board 210. In at least one embodiment, body feedback sensor(s) 250 are arranged in a manner similar to the stimulation components of stimulation component array 220. In alternative embodiments, body feedback sensor(s) 250 are disposed on or within flexible board 210 in alternative positions.

In embodiments such as that shown by stimulating device 200", body feedback sensor(s) 250 collect body feedback data which is provisioned to wireless radio 240. Thereafter, wireless radio 240 transmits body feedback data collected by at least one body feedback sensor(s) 250. Such information can be provided to, *e.g.,* a remote device.

The remote device, controller 230, or another component can respond to body feedback data in a variety of ways. Body feedback data can be logged for record-keeping or later analysis. In alternative or complementary embodiments, rule-based body feedback actions can be taken based on fixed or variable thresholds. For example, intensity can be increased or decreased based on increasing or decreasing pulse rates, or rates of change in pulse rates. Trend-based feedback can particularly evaluate rates of change in sensed body feedback data. In this regard, the stimulation signal can be modified or changed based on feedback. In an embodiment, vibration programs or schedules executed by a stimulation device can be selected for saving and/or "tuned" over time based on feedback from a user. For example, in the context of sexually stimulating devices, the identification of body feedback data indicating intense or multiple orgasms could be used to tailor stimulation signals according to the stimulation component inputs at the time of such body feedback data.

Further, statistical analysis can be performed on body feedback to determine individual or aggregated statistics related to body feedback independently or in conjunction with particular stimuli or patterns. Clusters of users who provide positive or negative feedback based on similar stimuli or patterns can be analyzed to tailor or tune stimulation signals for the group or to introduce new, modified, or popular stimulation signals to users having similar reactions as identified through statistical analysis. Such statistical analysis can be performed at, *e.g.,* a controller or a remote device. In an embodiment, recommendations are provided to a user using an external device or device in communication with a user account. Anomalies or rare reactions can also be monitored and tracked.

In still further embodiments, "games" or training programs can be created based on body feedback. In this regard, positive and negative feedback, including but not limited to increased or decreased energization of stimulating components, can be provided to a user based on whether actions taken are good or bad. In this manner, groups of users providing stimulation signals for one another can be rewarded for satisfying other users or performing in a preferred manner. Interactive media, games, workflows, training systems, and other arrangements can also be operatively coupled with stimulating components.

Controller 230 can include one or more microcontrollers integrated into the stimulating device. Alternatively or complementarily, microcontrollers can be integrated into a remote device. Wireless radio 240 can provide information, including body feedback data, to the microcontrollers for analysis or feedback activity.

Flexible sensors can be utilized to accord with the flexible board. Such sensors can be integrated into a circuit board or other components local to the user (or any other person or thing from which feedback is sought). These sensors can provide feedback and measurements to improve stimulation patterns or other performance. Data from sensors can be recorded and analyzed, provided real-time or in any other period to users, and/or implemented as modified control signals. For example, a user performing manual interactions can be provided feedback (*e*.*g*., graphically or otherwise) to assist or modify activity, or to aid in their modification of a pre-programmed control pattern.

Further, sensor data can be collected and compiled using network technologies. For example, sensor feedback can be returned to the Internet where user information and feedback can be compiled. Using machine learning, statistical analysis, user recommendations, *et cetera,* patterns and other actions can be improved, and recommendations provided.

Modifications to a stimulation signal can include engaging or disengaging one or more stimulating components, changing the timing of stimulation, changing the intensity of stimulation, rewarding or penalizing users, simulating activity not conducted with the board (*e*.*g*., coordinating action between multiple stimulating components to simulate motion in a static board), and so forth.

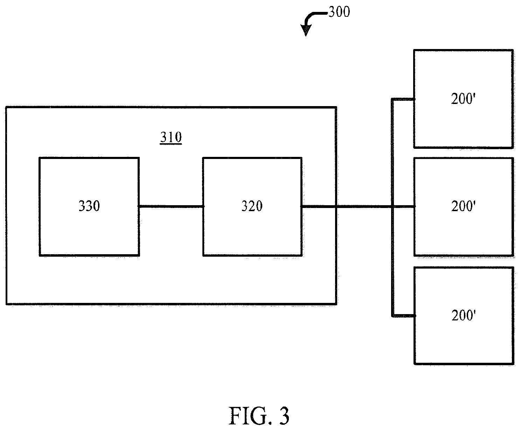

Fig. 3 illustrates a system 300 having a remote device 310 including a wireless radio 320 and a control module 330. Control module 330 can access and/or modify stimulation signals, and through operative coupling with wireless radio 320 provides one or more stimulation signals to two or more stimulation devices 200'. In this way, stimulation signals need not be controlled or modified in a one-to-one fashion, but can be broadcast to a plurality of users. In this way, group events and/or training relating to stimulation devices can be conducted. For example, a stimulation device use class can be used to demonstrate and teach multiple users at once regarding how to utilize or determine preferences for stimulation device 200'.

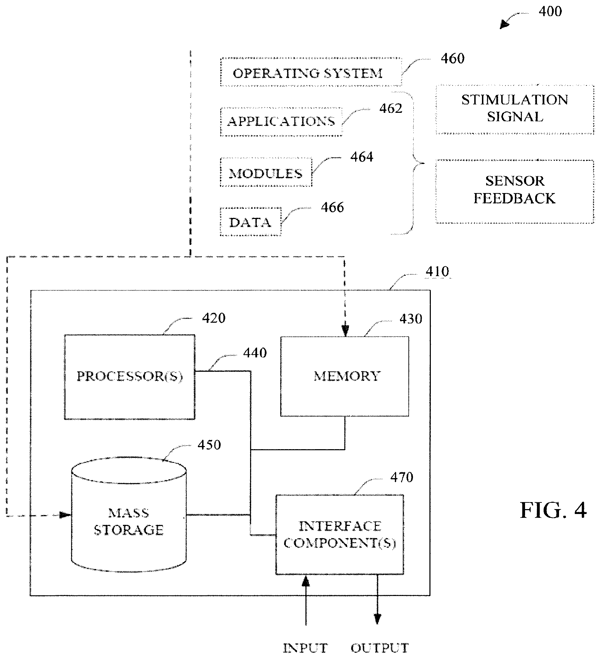

In order to provide a context for the claimed subject matter, Fig. 4 as well as the following discussion provide a brief, general description of a suitable environment in which various aspects of the subject matter can be implemented. This environment is only an example and is not intended to suggest any limitation as to scope of use or functionality.

While some of the above disclosed techniques can be described in the general context of computer-executable instructions of programs that runs on one or more computers or network hardware, those skilled in the art will recognize that aspects can also be implemented in combination with various alternative hardware, software, modules, et cetera. As suggested earlier, program modules and software components include routines, programs, components, data structures, among other things that perform particular tasks and/or implement particular abstract data types. Moreover, those skilled in the art will appreciate that the above systems and methods can be practiced with various computer system configurations, including single-processor, multi-processor or multi-core processor computer systems, mini-computing devices, mainframe computers, as well as personal computers, hand-held computing devices (*e*.*g*., personal digital assistant, portable gaming device, smartphone, tablet, Wi-Fi device, laptop, phone, among others), microprocessor-based or programmable consumer or industrial electronics, and the like. Aspects can also be practiced in distributed computing environments where tasks are performed by remote processing devices that are linked through a communications network. However, some, if not all aspects of the claimed subject matter can be practiced on stand-alone computers. In a distributed computing environment, program modules may be located in one or both of local and remote memory storage devices.

With reference to Fig. 4, illustrated is an example computer 410 or computing device (*e*.*g*., desktop, laptop, server, hand-held, programmable consumer or industrial electronics, set-top box, game system, *et cetera).* The computer 410 includes one or more processor(s) 420, memory 430, system bus 440, mass storage 450, and one or more interface components 470. The system bus 440 communicatively couples at least the above system components. However, it is to be appreciated that in its simplest form the computer 410 can include one or more processors 420 coupled to memory 430 that execute various computer executable actions, instructions, and or components stored in memory 430.

The processor(s) 420 can be implemented with a general purpose or specially manufactured processor, a digital signal processor (DSP), an application specific integrated circuit (ASIC), a field programmable gate array (FPGA) or other programmable logic device, discrete gate or transistor logic, discrete hardware components, or any combination thereof designed to perform the functions described herein. A general purpose processor may be a microprocessor, but in the alternative, the processor may be any processor, controller, microcontroller, or state machine. The processor(s) 420 may also be implemented as a combination of computing devices, for example a combination of a DSP and a microprocessor, a plurality of microprocessors, multi-core processors, one or more microprocessors in conjunction with a DSP core, or any other such configuration.

The computer 410 can include or otherwise interact with a variety of computer-readable media to facilitate control of the computer 410 to implement one or more aspects of the claimed subject matter. The computer-readable media can be any available media that can be accessed by the computer 410 and includes volatile and nonvolatile media, and removable and non-removable media. By way of example, and not limitation, computer-readable media may comprise computer storage media and communication media.

Computer storage media includes volatile and nonvolatile media, and removable and non-removable media, implemented in any method or technology for storage of information such as computer-readable instructions, data structures, program modules, or other data. Computer storage media includes, but is not limited to memory devices (*e*.*g*., random access memory, read-only memory, electrically erasable programmable read-only memory, *et cetera),* magnetic storage devices (*e*.*g*., hard disk, floppy disk, cassettes, tape, *et cetera*), optical disks (*e*.*g*., compact disk, digital versatile disk, et cetera), and solid state devices (*e*.*g*., solid state drive, flash memory drive such as a card, stick, or key drive, *et cetera*), or any other medium which can be used to store the desired information and which can be accessed by the computer 410.

Communication media typically embodies computer-readable instructions, data structures, program modules, or other data in a modulated data signal such as a carrier wave or other transport mechanism and includes any information delivery media. The term "modulated data signal" means a signal that has one or more of its characteristics set or changed in such a manner as to encode information in the signal. By way of example, and not limitation, communication media includes wired media such as a wired network or direct-wired connection, and wireless media such as acoustic, RF, infrared and other wireless media. Also, a connection can be a communication medium. For example, if the software is transmitted from a website, server, or other remote source using a coaxial cable, fiber optic cable, twisted pair, digital subscriber line (DSL), or wireless technologies such as infrared, radio, and microwave, then the coaxial cable, fiber optic cable, twisted pair, DSL, or wireless technologies such as infrared, radio and microwave are included in the definition of communication medium. Combinations of the above can also be included within the scope of computer-readable media.

Memory 430 and mass storage 450 are examples of computer-readable storage media. Depending on the exact configuration and type of computing device, memory 430 may be volatile *(e.g.,* RAM), non-volatile *(e.g.,* ROM, flash memory, et cetera) or some combination of the two. By way of example, the basic input/output system (BIOS), including basic routines to transfer information between elements within the computer 410, such as during start-up, can be stored in nonvolatile memory, while volatile memory can act as external cache memory to facilitate processing by the processor(s) 420, among other things.

Mass storage 450 includes removable/non-removable, volatile/non-volatile computer storage media for storage of large amounts of data relative to the memory 430. For example, mass storage 450 includes, but is not limited to, one or more devices such as a magnetic or optical disk drive, floppy disk drive, flash memory, solid-state drive, or memory stick.

Memory 430 and mass storage 450 can include, or have stored therein, operating system 460, one or more applications 462, one or more program modules 464, and data 466. The operating system 460 acts to control and allocate resources of the computer 410. Applications 462 include one or both of system and application software and can exploit management of resources by the operating system 460 through program modules 464 and data 466 stored in memory 430 and/or mass storage 450 to perform one or more actions. Accordingly, applications 462 can turn computer 410 into a specialized machine in accordance with the logic provided thereby.

All or portions of the claimed subject matter can be implemented using programming and/or engineering techniques to produce software, firmware, hardware, or any combination thereof to control a computer to realize the disclosed functionality. By way of example and not limitation, methodologies 500 and/or 600 can be, or form part of, an application 462, and include one or more modules 464 and data 466 stored in memory and/or mass storage 450 whose functionality can be realized when executed by one or more processor(s) 420.

In accordance with one particular embodiment, the processor(s) 420 can correspond to a system on a chip (SOC) or like architecture including, or in other words integrating, both hardware and software on a single integrated circuit substrate. Here, the processor(s) 420 can include one or more processors as well as memory at least similar to processor(s) 420 and memory 430, among other things. Conventional processors include a minimal amount of hardware and software and rely extensively on external hardware and software. By contrast, an SOC implementation of processor can be more powerful, as it embeds hardware and software therein that enable particular functionality with minimal or no reliance on external hardware and software. For example, instructions for methodologies 500 and/or 600 (and/or associated components) and can be embedded within hardware in a SOC architecture.

The computer 410 also includes one or more interface components 470 that are communicatively coupled to the system bus 440 and facilitate interaction with the computer 410. By way of example, the interface component 470 can be a port *(e.g.,* serial, parallel, PCMCIA, USB, FireWire, inter-integrated circuits, *et cetera)* or an interface card (*e.g.,* sound, video, *et cetera)* or the like. In one example implementation, the interface component 470 can be embodied as a user input/output interface to enable a user to enter commands and information into the computer 410 through one or more input devices (*e*.*g*., pointing device such as a mouse, trackball, stylus, touch pad, keyboard, microphone, joystick, game pad, satellite dish, scanner, camera, other computer, *et cetera).* In another example implementation, the interface component 470 can be embodied as an output peripheral interface to supply output to displays *(e.g.,* CRT, LCD, plasma, LED, *et cetera),* speakers, printers, and/or other computers, among other things. Still further yet, the interface component 470 can be embodied as a network interface to enable communication with other computing devices, such as over a wired or wireless communications link.

While aspects above are described at times as standalone or all-inclusive systems, it is understood that aspects herein can use the technology described above with various network elements *(e.g.,* servers, hubs, routers, *et cetera)* to accomplish multi-system or distributed network implementation of inventive techniques disclosed. Nothing herein should be construed as in any way limiting the network or distributive scope of embodiments embraced.

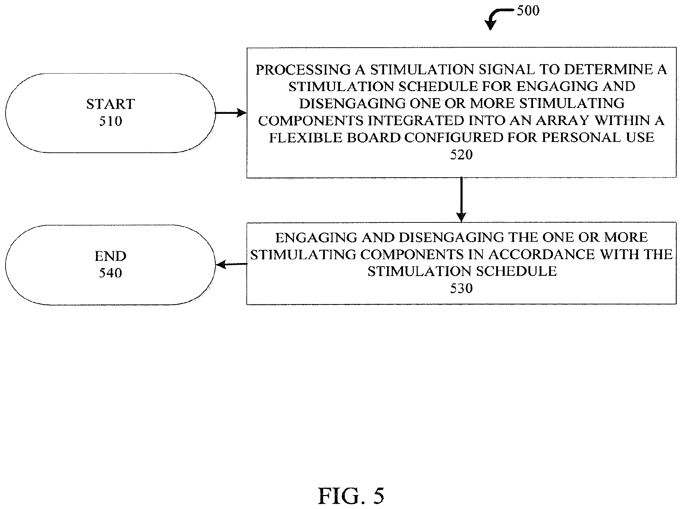

Turning to Fig. 5, methodology 500 provides a technique for engaging and disengaging stimulating components. At 510, methodology 500 beings, and proceeds to 520 where the methodology processes a stimulation signal to determine a stimulation schedule for engaging and disengaging one or more stimulating components integrated into an array within a flexible board configured for personal use. At 530, the methodology includes engaging and disengaging the one or more stimulating components in accordance with the stimulation schedule. After engaging or disengaging is complete, methodology 500 ends at 540.

In embodiments of methodology 500 (or other techniques described herein), the stimulation signal is received using a wireless radio operatively coupled with the flexible board. Where the wireless radio is a transceiver capable of sending and receiving information, feedback data from sensors can be provided back to the source of the stimulation signal (or a component operatively coupled therewith) for data collection or use as variables to modify the stimulation signal. In this regard, methods described herein can further include collecting body feedback data using at least one body feedback sensor integrated into the flexible board. The body feedback can be transmitted to other components or separate devices using the wireless radio.

Methods can include performing statistical analysis on the body feedback data to discern individual or aggregated trends related to stimulation or body feedback.

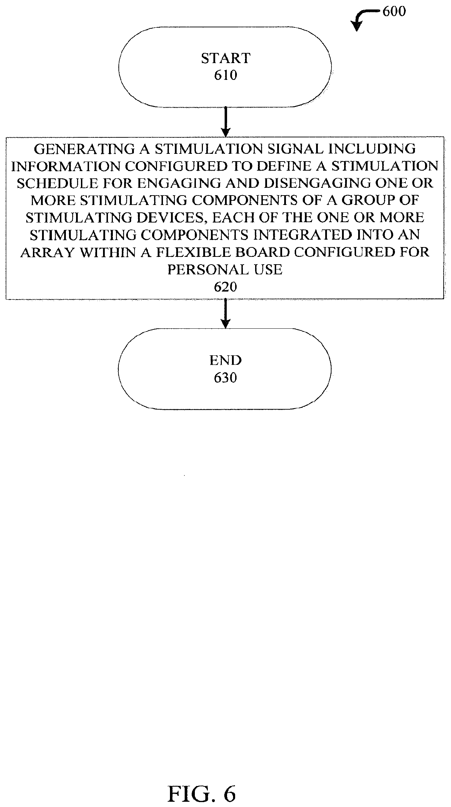

Fig. 6 illustrates a methodology 600 for providing a stimulation signal for a plurality of devices. Methodology 600 begins at 610 and proceeds to 620 which includes generating a stimulation signal including information configured to define a stimulation schedule for engaging and disengaging one or more stimulating components of a group of stimulating devices, each of the one or more stimulating components integrated into an array within a flexible board configured for personal use. Methodology 600 can thereafter end at 630.

Methodology 600 can further include engaging and disengaging the one or more stimulating components in at least one device among the group of stimulating devices according to the stimulation schedule. In still further embodiments, methodology 600 includes collecting body feedback data using at least one body feedback sensor integrated into the flexible board of at least one device among the group of stimulating devices. Can log or analyze. Analysis - analyzing the body feedback to produce a body feedback analysis result. If analyzed, modify the stimulation signal based on the body feedback analysis result.

Methodology 600 can be implemented using various components described in systems depicted herein. For example, Fig. 3 illustrates a "broadcast" technique where a single device sends stimulation signals to a plurality of stimulation devices.

Various embodiments of systems or methods herein can include additional aspects or elements improving functionality or convenience. Body feedback information provided by sensors or other components can be generated on a device such as a mobile phone. The mobile phone can be a remote device as described herein or distinct from such aspects. The mobile phone includes a custom app to recognize and control the device. In embodiments, the mobile phone additionally communicates with web based storage and programming. In such embodiments, the app may control the massaging/vibrating device(s) (*e*.*g*., by generating control signals, by processing feedback for use in control, by following templates or pre-recorded signals).

Additional functionality enabled by the mobile phone (or other processing device providing sending and receiving information to and from components acting on (or being acted on by) a user/wearer include the ability to load or share programs or tracks for the device, create or modify programs or customizations, and integrate media into programs or control routines.

Web based software can be configured to allow for the development and use of a variety of programs including games workflows that interact with one or many devices. Monitoring feedback to programs will provide for recommended improvements to programs, and track and find optimizations for increased stimulations or stress relief for an individual or groups. In embodiments, an open web based UI/SDK with media tracking can be employed.

In specific embodiments, the combined system can be realized as a remote massaging device for couples or groups. One or all participants can wear or establish contact with massaging/vibrating devices, which can have more than one area of possible activity. Vibration can be localized to a particular location or group of locations, and movement can be simulated through localization of such stimuli. The localized activity can reflect or simulate physical behavior conducted elsewhere, follow a predefined standard or custom stimulation schedule or plan, be provided in accordance with various selections (*e*.*g*., combinations of location and type of stimulation as selected from a menu or using prompts), or be provided according to various spontaneous or prompted movements in relation to a sensor or device receiving information about such. Various placement patterns for vibrators can be implemented and utilized to increase "resolution" as felt by the user.

Aspects herein can further be leveraged using manual control. Manual control can be provided or supplemented by various touchscreens and user interfaces including graphical overlays of pictures, animations, video, *et cetera.* Pattern interactive programming can be provided to teach patterns or produced enhanced patterns based on feedback or other behavior (*e.g*., as a "reward"). In this way, system control can be "game-ified."

In wearable embodiments, various means of integration can be utilized. A harness can be embedded within or worn under clothing. Alternatively, a contoured shell or other structure can be placed and retained by other clothing or the body on which it is worn. For example, a flexible circuit board can be coated in silicone to establish a pad which can be attached to or placed within clothing.

In the specification and claims, reference is made to a number of terms that have the following meanings. The singular forms "a", "an" and "the" include plural referents unless the context clearly dictates otherwise. Approximating language, as used herein throughout the specification and claims, may be applied to modify a quantitative representation that could permissibly vary without resulting in a change in the basic function to which it is related. Accordingly, a value modified by a term such as "about" is not to be limited to the precise value specified. In some instances, the approximating language may correspond to the precision of an instrument for measuring the value. Moreover, unless specifically stated otherwise, a use of the terms "first," "second," etc., do not denote an order or importance, but rather the terms "first," "second," etc., are used to distinguish one element from another.

As used herein, the terms "may" and "may be" indicate a possibility of an occurrence within a set of circumstances; a possession of a specified property, characteristic or function; and/or qualify another verb by expressing one or more of an ability, capability, or possibility associated with the qualified verb. Accordingly, usage of "may" and "may be" indicates that a modified term is apparently appropriate, capable, or suitable for an indicated capacity, function, or usage, while taking into account that in some circumstances the modified term may sometimes not be appropriate, capable, or suitable. For example, in some circumstances an event or capacity can be expected, while in other circumstances the event or capacity cannot occur - this distinction is captured by the terms "may" and "may be."

As utilized herein, the term "or" is intended to mean an inclusive "or" rather than an exclusive "or." That is, unless specified otherwise, or clear from the context, the phrase "X employs A or B" is intended to mean any of the natural inclusive permutations. That is, the phrase "X employs A or B" is satisfied by any of the following instances: X employs A; X employs B; or X employs both A and B. In addition, the articles "a" and "an" as used in this application and the appended claims should generally be construed to mean "one or more" unless specified otherwise or clear from the context to be directed to a singular form.

The invention is set out in the following claims.

## Claims

1. A stimulation device (200, 200', 200"), comprising:
a flexible board (210) configured for personal use;
an array of one or more vibrational stimulating components (220) disposed throughout at least a portion of the flexible board;
a stimulation controller (230) in communication with at least one of the one or more vibrational stimulating components, the stimulation controller configured to process a stimulation signal that activates or deactivates at least one of the one or more vibrational stimulating components;
at least one sensor (250) integrated into the flexible board for collecting body feedback data; and
a wireless radio (240) in communication with the stimulation controller (230) and configured to receive the stimulation signal wirelessly from at least one remote device,
wherein the controller is further configured to modify the stimulation signal based on the body feedback data.

2. The stimulation device (200, 200', 200") of claim 1, wherein the wireless radio (240) is configured to transmit the body feedback data collected by the at least one body feedback sensor (250) to the at least one remote device.

3. The stimulation device (200, 200', 200") of claim 1, wherein the flexible board (210) is formed at least in part of a flexible conductive material.

4. The stimulation device (200, 200', 200") of claim 1, wherein the flexible board (210) is formed into a ring or cylinder.

5. The stimulation device (200, 200', 200") of claim 1, wherein at least one of the one or more vibrational stimulating components (220) is configured to activate at varying intensity.

6. A non-therapeutic method, comprising:
processing a stimulation signal that activates one or more vibrational stimulating components (220) integrated into an array within a flexible board (210) configured for personal use;
activating and deactivating the one or more vibrational stimulating components;
collecting body feedback data using at least one body feedback sensor (250) integrated into the flexible board; and
modifying the stimulation signal based on the body feedback data,
wherein the stimulation signal is received using a wireless radio (240) operatively coupled with the flexible board (210).

7. The method of claim 6, further comprising:
transmitting the body feedback data using the wireless radio (240).

8. The method of claim 6, wherein the flexible board (210) is formed at least in part of a flexible conductive material.

9. The method of claim 6, wherein the flexible board (210) is formed into a ring or cylinder.

10. The method of claim 6, wherein the array of one or more vibrational stimulating components (220) is a three dimensional array.

11. The method of claim 6, further comprising:
generating a stimulation signal including information configured to define a stimulation schedule for activating and deactivating one or more vibrational stimulating components (220) of a group of stimulating devices (200, 200', 200"), each of the one or more vibrational stimulating components integrated into an array within a flexible board (210) configured for personal use; and
collecting body feedback data using at least one body feedback sensor (250) integrated into the flexible board of at least one device among the group of stimulating devices.

12. The method of claim 11, further comprising activating and deactivating the one or more vibrational stimulating components (220) in at least one device among the group of stimulating devices (200, 200', 200") according to the stimulation schedule.

13. The method of claim 12, further comprising:
analyzing the body feedback to produce a body feedback analysis result; and
modifying the stimulation signal based on the body feedback analysis result.

## Patentansprüche

1. Stimulationsvorrichtung (200, 200', 200"), umfassend:
eine flexible Platine (210), konfiguriert für den persönlichen Gebrauch;
ein Array aus einer oder mehreren vibrationsstimulierenden Komponenten (220), die über mindestens einen Abschnitt der flexiblen Platine verteilt sind;
eine Stimulationssteuerung (230), die mit mindestens einer der einen oder mehreren vibrationsstimulierenden Komponenten in Kommunikation steht, wobei die Stimulationssteuerung konfiguriert ist, um ein Stimulationssignal zu verarbeiten, das mindestens eine der einen oder mehreren vibrationsstimulierenden Komponenten aktiviert oder deaktiviert;
mindestens einen Sensor (250), der in die flexible Platine integriert ist, um Körperrückmeldedaten zu sammeln; und
ein drahtloses Funkgerät (240), das mit der Stimulationssteuerung (230) in Kommunikation steht und konfiguriert ist, um das Stimulationssignal drahtlos von mindestens einer entfernten Vorrichtung zu empfangen, wobei die Steuerung ferner konfiguriert ist, das Stimulationssignal basierend auf den Körperrückmeldedaten zu modifizieren.

2. Stimulationsvorrichtung (200, 200', 200") nach Anspruch 1, wobei das drahtlose Funkgerät (240) konfiguriert ist, um die von dem mindestens einen Körperrückmeldesensor (250) erfassten Körperrückmeldedaten an die mindestens eine entfernte Vorrichtung zu übertragen.

3. Stimulationsvorrichtung (200, 200', 200") nach Anspruch 1, wobei die flexible Platine (210) mindestens teilweise aus einem flexiblen leitfähigen Material ausgebildet ist.

4. Stimulationsvorrichtung (200, 200', 200") nach Anspruch 1, wobei die flexible Platine (210) zu einem Ring oder Zylinder ausgebildet ist.

5. Stimulationsvorrichtung (200, 200', 200") nach Anspruch 1, wobei mindestens eine der einen oder mehreren vibrationsstimulierenden Komponenten (220) konfiguriert ist, mit unterschiedlicher Intensität aktiviert zu werden.

6. Nicht-therapeutisches Verfahren, umfassend:
Verarbeiten eines Stimulationssignals, das eine oder mehrere vibrationsstimulierende Komponenten (220) aktiviert, die in einem Array innerhalb einer flexiblen Platine (210) integriert sind, die für den persönlichen Gebrauch konfiguriert ist;
Aktivieren und Deaktivieren der einen oder mehreren vibrationsstimulierenden Komponenten;
Sammeln von Körperrückmeldedaten unter Verwendung mindestens eines Körperrückmeldesensors (250), der in die flexible Platte integriert ist; und
Modifizieren des Stimulationssignals basierend auf den Körperrückmeldedaten, wobei das Stimulationssignal unter Verwendung eines drahtlosen Funkgeräts (240) empfangen wird, das operativ mit der flexiblen Platine (210) gekoppelt ist.

7. Verfahren nach Anspruch 6, ferner umfassend:
Übertragen der Körperrückmeldedaten unter Verwendung des drahtlosen Funkgeräts (240).

8. Verfahren nach Anspruch 6, wobei die flexible Platine (210) mindestens teilweise aus einem flexiblen leitfähigen Material ausgebildet ist.

9. Verfahren nach Anspruch 6, wobei die flexible Platine (210) zu einem Ring oder Zylinder ausgebildet ist.

10. Verfahren nach Anspruch 6, wobei das Array aus einer oder mehreren vibrationsstimulierenden Komponenten (220) ein dreidimensionales Array ist.

11. Verfahren nach Anspruch 6, ferner umfassend:
Erzeugen eines Stimulationssignals, das Informationen beinhaltet, die konfiguriert sind, um einen Stimulationsplan zum Aktivieren und Deaktivieren einer oder mehrerer vibrationsstimulierender Komponenten (220) einer Gruppe von Stimulationsvorrichtungen (200, 200', 200") zu definieren, wobei jede der einen oder mehreren vibrationsstimulierenden Komponenten in ein Array innerhalb einer flexiblen Platine (210) integriert ist, die für den persönlichen Gebrauch konfiguriert ist; und
Sammeln von Körperrückmeldedaten unter Verwendung mindestens eines Körperrückmeldesensors (250), der in die flexible Platine mindestens einer Vorrichtung aus der Gruppe der Stimulationsvorrichtungen integriert ist.

12. Verfahren nach Anspruch 11, ferner umfassend Aktivieren und Deaktivieren der einen oder mehreren vibrationsstimulierenden Komponenten (220) in mindestens einer Vorrichtung aus der Gruppe der Stimulationsvorrichtungen (200, 200', 200") gemäß dem Stimulationsplan.

13. Verfahren nach Anspruch 12, ferner umfassend:
Analysieren der Körperrückmeldung, um ein Ergebnis der Körperrückmeldeanalyse zu erzeugen; und
Modifizieren des Stimulationssignals basierend auf dem Ergebnis der Körperrückmeldeanalyse.

## Revendications

1. Dispositif de stimulation (200, 200', 200"), comprenant :
une carte souple (210) conçue pour un usage personnel ;
un réseau d'un ou de plusieurs composants de stimulation vibratoire (220) disposés sur au moins une partie de la carte souple ;
un dispositif de commande de stimulation (230) en communication avec au moins l'un des un ou plusieurs composants de stimulation vibratoire, le dispositif de commande de stimulation étant conçu pour traiter un signal de stimulation qui active ou désactive au moins l'un des un ou plusieurs composants de stimulation vibratoire ;
au moins un capteur (250) intégré dans la carte souple pour collecter des données de rétroaction corporelle ; et
une radio sans fil (240) en communication avec le dispositif de commande de stimulation (230) et conçue pour recevoir sans fil le signal de stimulation en provenance d'au moins un dispositif à distance, dans lequel le dispositif de commande est en outre conçu pour modifier le signal de stimulation sur la base des données de rétroaction corporelles.

2. Dispositif de stimulation (200, 200', 200") selon la revendication 1, dans lequel la radio sans fil (240) est conçue pour transmettre les données de rétroaction corporelle collectées par l'au moins un capteur de rétroaction corporelle (250) à l'au moins un appareil à distance.

3. Dispositif de stimulation (200, 200', 200") selon la revendication 1, dans lequel la carte souple (210) est formée au moins en partie d'un matériau conducteur souple.

4. Dispositif de stimulation (200, 200', 200") selon la revendication 1, dans lequel la carte souple (210) est formée en un anneau ou un cylindre.

5. Dispositif de stimulation (200, 200', 200") selon la revendication 1, dans lequel au moins l'un des un ou plusieurs composants de stimulation vibratoire (220) est conçu pour s'activer à une intensité variable.

6. Procédé non thérapeutique, comprenant :
le traitement d'un signal de stimulation qui active un ou plusieurs composants de stimulation vibratoire (220) intégrés dans un réseau à l'intérieur d'une carte souple (210) conçue pour un usage personnel ;
l'activation et la désactivation des un ou plusieurs composants de stimulation vibratoire ;
la collecte de données de rétroaction corporelle à l'aide d'au moins un capteur de rétroaction corporelle (250) intégré dans la carte souple ; et
la modification du signal de stimulation sur la base des données de rétroaction du corps, dans lequel le signal de stimulation est reçu à l'aide d'une radio sans fil (240) couplée de manière fonctionnelle à la carte souple (210).

7. Procédé selon la revendication 6, comprenant en outre :
la transmission des données de rétroaction corporelle à l'aide de la radio sans fil (240).

8. Procédé selon la revendication 6, dans lequel la carte souple (210) est formée au moins en partie d'un matériau conducteur souple.

9. Procédé selon la revendication 6, dans lequel la carte souple (210) est formée en un anneau ou un cylindre.

10. Procédé selon la revendication 6, dans lequel le réseau d'un ou de plusieurs composants de stimulation vibratoire (220) est un réseau tridimensionnel.

11. Procédé de la revendication 6, comprenant également :
la génération d'un signal de stimulation comprenant des informations conçues pour définir un programme de stimulation pour activer et désactiver un ou plusieurs composants de stimulation vibratoire (220) d'un groupe de dispositifs de stimulation (200, 200', 200''), chacun des un ou plusieurs composants de stimulation vibratoire étant intégré dans un réseau à l'intérieur d'une carte souple (210) conçue pour un usage personnel ; et
la collecte de données de rétroaction corporelle à l'aide d'au moins un capteur de rétroaction corporelle (250) intégré dans la carte souple d'au moins un dispositif parmi le groupe de dispositifs de stimulation.

12. Procédé selon la revendication 11, comprenant en outre l'activation et la désactivation des un ou plusieurs composants de stimulation vibratoire (220) dans au moins un dispositif parmi le groupe de dispositifs de stimulation (200, 200', 200") selon le programme de stimulation.

13. Procédé selon la revendication 12, comprenant en outre :
l'analyse de la rétroaction corporelle pour produire un résultat d'analyse de rétroaction corporelle ; et
la modification du signal de stimulation sur la base du résultat d'analyse de rétroaction corporelle.
